# EUROPEAN PATENT SPECIFICATION

(11) **EP 3 078 320 B1**
(45) Date of publication and mention of the grant of the patent: **12.09.2018**
(21) Application number: 15835602.2
(22) Date of filing: 16.07.2015
(51) Int. Cl.: A61B 1/00, G02B 23/24, A61M 25/01

(54) **WIRE DEVICE AND ENDOSCOPE**
DRAHTVORRICHTUNG UND ENDOSKOP
DISPOSITIF À FIL MÉTALLIQUE ET ENDOSCOPE

(30) Priority: 27.08.2014 JP 2014172988
(43) Date of publication of application: 12.10.2016
(73) Proprietor: Olympus Corporation, Tokyo 192-8507 (JP)
(72) Inventor: MIYAGI, Masaaki, Hachioji-shi, Tokyo 192-8507 (JP); OKAZAKI, Tsugio, Hachioji-shi, Tokyo 192-8507 (JP); SHIGA, Naohito, Hachioji-shi, Tokyo 192-8507 (JP)
(74) Representative: Gunzelmann, Rainer
(86) International application number: PCT/JP2015/070358
(87) International publication number: WO 2016/031419

(56) References cited:
- JP-A- S62 134 616
- JP-A- 2000 166 858
- JP-A- 2014 033 933
- US-A- 5 752 912
- US-A1- 2013 137 928

## Description

### Technical Field

The present invention relates to a wire apparatus and an endoscope.

### Background Art

In general, an endoscope includes an elongated insertion portion to be inserted into a region to be examined and an operation portion coupled to a proximal end side of the insertion portion. The insertion portion includes a flexible tube portion which is elongated and has flexibility, a bending portion disposed on a distal end side of the flexible tube portion, and a distal end rigid portion disposed on the distal end side of the bending portion. The operation portion is provided with a bending operation device for performing a bending operation of the bending portion.

Inside the insertion portion, at least a pair of metal bending operation wires is provided so as to extend in a longitudinal direction. The bending operation wire moves forward or backward in accordance with the bending operation performed by the bending operation device. One of the pair of the bending operation wires moves to the proximal end side and the other of the pair of the bending operation wires moves to the distal end side by the bending operation performed by the bending operation device, to thereby cause the bending portion to bend.

Inside the flexible tube portion, the same number of coil pipes as that of the bending operation wires are provided so as to extend in the longitudinal direction. The coil pipes are guide members that guide the bending operation wires. In each of the coil pipes, one corresponding bending operation wire of the bending operation wires is inserted so as to be able to move forward or backward.

In addition, the bending portion includes inside thereof a bending piece group formed by rotatably coupling a plurality of bending pieces with each other in an elongated shape in the longitudinal direction. The respective bending pieces are provided with the same number of wire receivers as that of the bending operation wires, for example. One corresponding operation wire of the bending operation wires is inserted through each of the wire receivers so as to be able to move forward or backward.

When an insertion portion of a large intestine endoscope is inserted into the large intestine, the flexible tube portion of the insertion portion deforms in a complicated shape such as a loop shape. In such a deformed state, if a bending operation wire is moved in the longitudinal direction by performing the bending operation with the bending operation device, a frictional force is generated between the outer circumferential surface of the bending operation wire and the inner circumferential surface of the coil pipe, which increases sliding resistance. The amount of operation force at the time when the bending operation device is operated for the bending operation becomes large as the sliding resistance becomes large.

Meanwhile, when the bending portion is bent by performing bending operation with the bending operation device, the bending operation wire receives a load from the wire receiver, and the like. When the load received by the bending operation wire is large, there is a possibility of occurrence of such a failure that the bending operation wire is cut.

In order to reduce the amount of operation force at the time of bending operation and prevent a large load from being applied to the bending operation wire, an endoscope is configured such that lubricating coating is applied to the outer circumferential surface of the bending operation wire or powder solid lubricant is adhered to the outer circumferential surface of the bending operation wire.

However, the powder as the solid lubricant adhered to the outer circumferential surface of the bending operation wire easily falls off from the wire by the sliding of the wire in the longitudinal direction.

For example, Japanese Patent Application Laid-Open Publication No. 2014-033933 discloses an endoscope configured such that the coating part of the bending operation wire is resistant to low-temperature plasma sterilization and capable of reducing the amount of operation force at the time of bending operation without increasing the inner diameter and the outer diameter of the coil pipe and effectively preventing the bending operation wire from being cut in the bending portion. In this endoscope, the coating part including a first film-forming portion and a second film-forming portion is provided on the outer circumferential surface of the bending operation wire.

However, there is a possibility that the coating part applied on the outer circumferential surface of the bending operation wire is peeled off from the wire by the bending operation wire sliding in the coil pipe or contacting the wire receiver.

If the coating part, which includes a binder, of the bending operation wire is peeled off from the operation wire in the inner portion of the coil pipe, which is narrow space, there is a possibility that the binder included in the peeled-off coating part adheres to the inner surface of the coil pipe and interferes with the sliding of the bending operation wire.

In addition, the coating part applied to the outer circumferential surface of a wire configured to be inserted through the sheath, such as a forceps raising wire, a lens operation wire, an insertion assisting portion driving force transmitting wire, or the like, is likely to be peeled off from the wire by the sliding of the wire in the sheath.

On the other hand, if the coating part, which includes the binder, of the bending operation wire is peeled off from the operation wire in the bending piece group as a large space, the peeled-off coating part and the binder are scattered in the bending piece group. In this case, the binder and the coating including the binder scattered in the bending piece group cannot be a cause for interfering with the sliding of the bending operation wire.

Note that when the powder of the solid lubricant adhered to the outer circumferential surface of the bending operation wire falls off in the bending piece group, the power is scattered in the bending piece group and after that, it is not likely that the powder adheres to the same position of the bending operation wire again.

The present invention has been achieved in view of the above-described circumstances, an object of the present invention is to provide a bending apparatus configured to reduce an amount of operation force at the time of bending operation and prevent a bending operation wire from being cut in a bending portion.

### Disclosure of Invention

### Means for Solving the Problem

A wire apparatus according to one aspect of the present invention includes: a sheath arranged inside an endoscope; and a wire inserted in the sheath and moved in an axial direction or rotated in the sheath, wherein the wire is configured such that processing for coating including a binder component is performed only on a range of the wire, the range being exposed from the sheath, and a powder lubricant which does not include the binder component is applied to at least a range of the wire, the range being inserted in the sheath.

An endoscope according to one aspect of the present invention includes a wire apparatus including a sheath arranged inside an endoscope, and a wire inserted in the sheath and moved in an axial direction or rotated in the sheath, wherein the wire is configured such that processing for coating including a binder component is performed only on a range of the wire, the range being exposed from the sheath, and a powder lubricant which does not include the binder component is applied to at least a range of the wire, the range being inserted in the sheath.

### Brief Description of the Drawings

FIG. 1 illustrates an endoscope according to an embodiment of the present invention.
FIG. 2 is a schematic view illustrating an inner structure of the endoscope.
FIG. 3 is a cross-sectional view illustrating configurations of a corrugated tube portion and a bending portion of the endoscope.
FIG. 4 is a cross-sectional view taken along Y4-Y4 line in FIG. 3.
FIG. 5 is a cross-sectional view taken along Y5-Y5 line in FIG. 3.

### Best Mode for Carrying Out the Invention

Hereinafter, description will be made on the present invention with reference to the embodiment shown in the drawings.

In the drawings used in the description below, there is a case where a different scale size is used for each of the constituent elements in order to allow each of the constituent elements to be illustrated in a recognizable size in the drawings. Therefore, in the present invention, the number, shapes, ratio of the sizes of the constituent elements, and a relative positional relationship among the constituent elements shown in these drawings are not limited to the embodiment shown in the drawings.

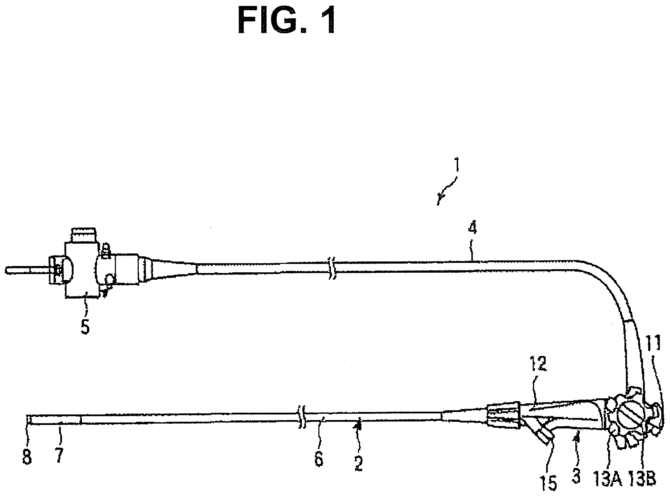

With reference to FIGS. 1 to 5, an embodiment of the present invention will be described.

As shown in FIG. 1, an endoscope 1 is a bending apparatus and includes an elongated insertion portion 2 to be inserted into a body cavity which is a region to be examined, for example, and an operation portion 3 coupled to the proximal end side of the insertion portion 2. A universal cord 4 is connected to the proximal end side of the operation portion 3. A scope connector 5 is provided at the proximal end portion of the universal cord 4.

The insertion portion 2 includes an elongated and flexible corrugated tube portion 6 as a tube, a bending portion 7, which is a bending tube configured to be bendable and which is coupled to the distal end side of the corrugated tube 6, and a distal end rigid portion 8 provided on the distal end side of the bending portion 7. The corrugated tube portion 6 has flexibility and is bent by an external force being applied thereto. The distal end rigid portion 8 is provided with an image pickup device (not shown) that picks up an image of an object, an air/water feeding nozzle (not shown), and the like. Note that the tube is not limited to the elongated and flexible corrugated tube portion, and may be a rigid and elongated tube portion.

The operation portion 3 includes an operation portion casing 11, and a holding portion casing 12. The operation portion casing 11 is provided with an up/down bending operation knob 13A that performs bending operation of the bending portion 7 in up and down directions and a right/left bending operation knob 13B that performs bending operation of the bending portion 7 in right and left directions.

The holding portion casing 12 is provided on the insertion portion 2 side with respect to the operation portion casing 11 and includes a forceps port 15.

Hereinafter, description will be made on an up/down bending operation mechanism for bending the bending portion 7 in the up and down directions.

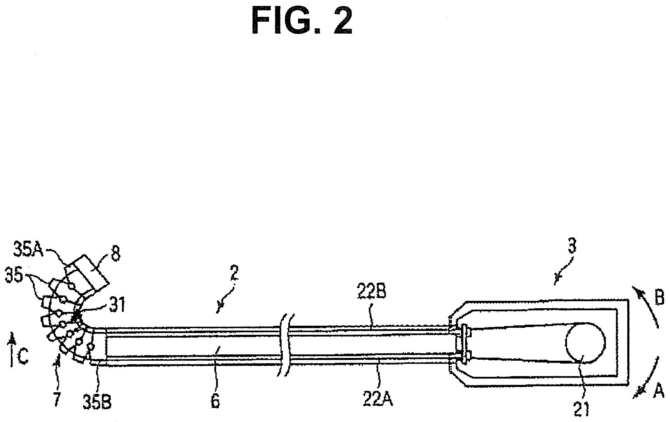

As shown in FIG. 2, the operation portion 3 includes inside thereof a pulley 21 as a rotation member, for example. The pulley 21 is coupled to the up/down bending operation knob 13A. The pulley 21 rotates in the direction of the arrow A or in the direction of the arrow B in FIG. 2 in accordance with the bending operation by the up/down bending operation knob 13A. The pulley 21 is connected with one end of each of a pair of bending operations wires 22A, 22B that are bending operation transmitting members configured to transmit the bending operation by the up/down bending operation knob 13A to the bending portion 7.

The bending portion 7 includes a bending piece group 31. The bending piece group 31 is formed by rotatably coupling a plurality of bending pieces 35 in the longitudinal direction. Each of the bending operation wires 22A, 22B is inserted through inside the corrugated tube portion 6 and inside the bending portion 7 along the longitudinal axis of the insertion portion 2.

The other end of each of the bending operation wires 22A, 22B is connected at a previously determined position of a distal end bending piece 35A arranged on the distal most side of the bending piece group 31. That is, each of the bending operation wires 22A, 22B passes through the inside of the operation portion 3 and the inside of the corrugated tube portion 6, to be extended to the inside of the bending portion 7.

When the pulley 21 is rotated in the direction of the arrow A in FIG. 2 by operating the up/down bending operation knob 13A, the one bending operation wire 22A moves to the distal end side, and the other bending operation wire 22B moves to the proximal end side. This causes the bending portion 7 to bend in the direction of the arrow C (up direction) in FIG. 2. Conversely, when the pulley 21 is rotated in the direction of the arrow B in FIG. 2 by operating the up/down bending operation knob 13A, the bending operation wire 22B moves to the distal end side and the bending operation wire 22A moves to the proximal end side. This causes the bending portion 7 to bend in the direction opposite to the arrow C (down direction) in FIG. 2.

Thus, the up/down bending operation mechanism that causes the bending portion 7 to bend in the up and down directions is configured.

Note that the right/left bending operation mechanism that causes the bending portion 7 to bend in the right and left directions has the same configuration as that of the up/down bending operation mechanism. Therefore, the description of the right/left bending operation mechanism will be omitted.

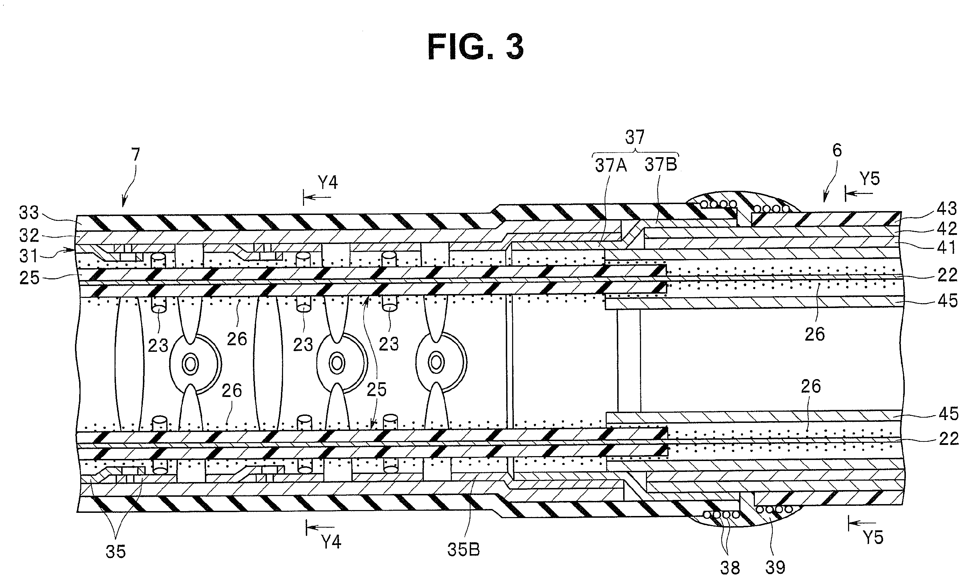

As shown in FIG. 3, the corrugated tube portion 6 includes, in a layered manner, a spiral tube 41 made of metal, a corrugated tube portion mesh tube 42 provided on the outer circumferential side of the spiral tube 41, and a corrugated tube outer cover 43 which is made of resin and which covers the outer circumferential surface of the corrugated tube portion mesh tube 42. The spiral tube 41 is formed by spirally winding a metal band-like member at predetermined intervals.

The bending portion 7 includes, in a layered manner, the above-described bending piece group 31, a bending portion mesh tube 32 provided on the outer circumferential side of the bending piece group 31, and a bending portion outer cover 33 which is made of rubber and which covers the outer circumferential surface of the bending portion mesh tube 32. The corrugated tube portion 6 and the bending portion 7 are configured by coupling a connecting pipe sleeve 37 of the corrugated tube portion 6 with a proximal end bending piece 35B arranged on the proximal-most side of the bending piece group 31.

The connecting pipe sleeve 37 includes a small-diameter portion 37A which has a substantially same diameter as that of the spiral tube 41, and a large-diameter portion 37B which is provided on the proximal end side of the small-diameter portion 37A and which has a diameter larger than that of the small-diameter portion 37A, for example. The proximal end bending piece 35B is disposed so as to be externally fitted to the small-diameter portion 37A of the connecting pipe sleeve 37. The large-diameter portion 37B of the connecting pipe sleeve 37 is disposed so as to be externally fitted to the spiral tube 41 and the corrugated tube mesh tube 42. Thus, the corrugated tube portion 6 and the bending portion 7 are coupled with each other. In addition, in the part around a the distal end portion of the corrugated tube outer cover 43 and the proximal end portion of the bending portion outer cover 33, a thread 38 is wound around the corrugated tube outer cover 43 and the bending portion outer cover 33, and thereafter an adhesive 39 is applied to the part.

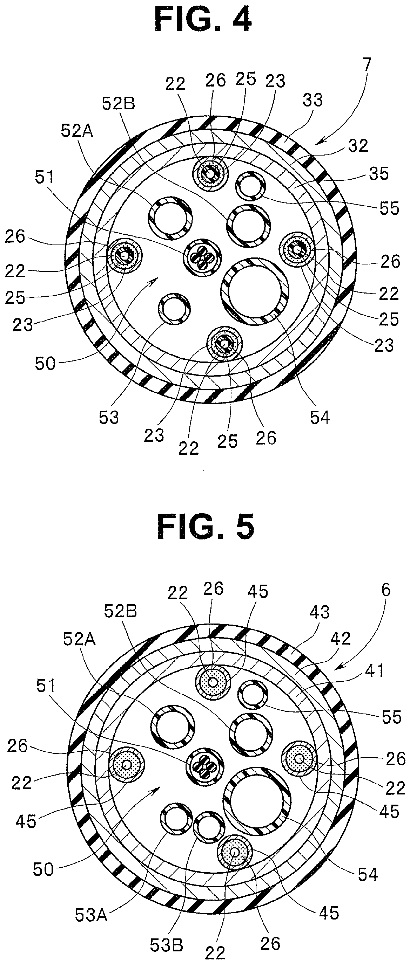

As shown in FIG. 4 and FIG. 5, inside the corrugated tube portion 6 and bending portion 7, various kinds of long and flexible internal components 50 such as an image pickup cable 51, a light guide 52, an air/water feeding tube 53, a forceps tube 54, an auxiliary water feeding tube 55, and the like are inserted.

The distal end portion of the image pickup cable 51 is connected to an image pickup device (not shown) in the distal end rigid portion 8. The proximal end portion of the image pickup cable 51 passes through the operation portion 3 and the universal cord 4, to be connected to an image observation apparatus (not shown) through a scope connector 5.

The proximal end portion of the light guide 52 passes through the operation portion 3 and the universal cord 4, to be connected to an illumination power source apparatus (not shown) through the scope connector 5. The light guide 52 guides the light for illuminating the object to an illumination window (not shown) of the distal end rigid portion 8.

The distal end portion of the air/water feeding tube 53 is coupled with the air/water feeding channel (not shown) of the distal end rigid portion 8. The air/water feeding tube 53 is branched off to an air feeding tube 53A and a water feeding tube 53B inside the corrugated tube portion 6.

The proximal end portion of the air feeding tube 53A passes through the operation portion 3 and the universal cord 4, to be connected to an air feeding apparatus (not shown) through the scope connector 5. The proximal end portion of the water feeding tube 53B passes through the operation portion 3 and the universal cord 4 to be connected to a water feeding apparatus (not shown) through the scope connector 5.

The distal end portion of the forceps tube 54 is coupled with a forceps channel (not shown) of the distal end rigid portion 8. In addition, the forceps tube 54 is bifurcated inside the operation portion 3, and one of the bifurcated portions is connected to the forceps port 15. The other of the bifurcated portions passes through the universal cord 4 to be connected to a suction apparatus (not shown) through the scope connector 5.

The distal end portion of the auxiliary water feeding tube 55 is coupled with an auxiliary water feeding channel (not shown) of the distal end rigid portion 8. The proximal end portion of the auxiliary water feeding tube 55 passes through the operation portion 3 and the universal cord 4, to be connected to another water feeding apparatus (not shown) different from the water feeding apparatus that feeds water to the air/water feeding tube 53, through the scope connector 5.

As shown in FIG. 3 and FIG. 5, four coil pipes 45 are arranged inside the corrugated tube portion 6, as four sheaths corresponding respectively to bending directions. Each of the coil pipes 45 is formed by spirally winding an element wire and is a closed coil in which adjacent turns of the element wire contact with one another in a natural state. The sheaths are made of resin, for example.

The distal end portions of the four coil pipes 45 are attached to previously determined positions of the connecting pipe sleeve 37 by soldering, for example, and arranged in the corrugated tube portion 6. Inside each of the coil pipes 45, one corresponding bending operation wire 22, which is among the bending operation wires 22A, 22B that transmit the bending operation in the up and down directions, and bending operation wires 22C, 22D that transmit the bending operation in the right and left directions (hereinafter, bending operation wires 22A, 22B, 22C, and 22D are referred to as the bending operation wires 22 except in a predetermined case), is inserted so as to be able to move forward or backward along the longitudinal direction. That is, each of the coil pipes 45 is a guide member configured by spirally winding the element wire around each of the operation wires, and one corresponding bending operation wire 22 among the bending operation wires 22 is inserted in each of the coil pipes 45.

As shown in FIG. 3 and FIG. 4, the bending operation wires 22 are extended respectively from the distal end portions of the coil pipes 45 further to the distal end side than the connecting pipe sleeve 37. The four wire receivers 23 respectively corresponding to the bending directions are provided on the inner circumferential surface of each of the bending pieces 35 constituting the bending piece group 31.

The wire receivers 23 are arranged respectively at 90-degree intervals in the circumferential direction of the insertion portion 2. Each of the wire receivers 23 is a support portion having a through hole through which one bending operation wire 22 is inserted. One corresponding bending operation wire 22 among the four bending operation wires 22 is arranged in each of the wire receivers 23.

The distal end portion of each of the bending operation wires 22 is fixed to a wire stopper (not shown) provided at the distal end bending piece 35A. The bending portion 7 is bent as described above by pulling or relaxing each of the bending operation wires 22 in the longitudinal direction of the insertion portion 2.

As shown in FIG. 3, on a part of the outer circumferential surface of each of the bending operation wires 22, a coating portion 25 of a predetermined thickness formed by coating processing is provided. The coating portion 25 is made of a material such as graphite-containing fluorine resin coating, PTFE (polytetrafluoroethylene)-nickel plating, PTFE-epoxy resin coating including epoxy resin and phenol resin as binder components.

The coating portion 25 is provided on a range, which is located inside the bending portion 7, of each of the bending operation wires 22. In other words, the coating portion 25 is not provided on a range, which is inserted in each of the coil pipes 45 provided inside the corrugated tube portion 6, of each of the bending operation wires 22.

That is, the coating portion 25 of each of the bending operation wires 22 is provided at the part contacting each of the wire receivers 23.

In the present embodiment, a powder lubricant (powder of the above-described solid lubricant) 26 is applied to the range of each of the bending operation wires 22 where the coating portion 25 is provided and to the range where the coating portion 25 is not provided (bare part).

Description will be made on the working of the endoscope 1 according to the present embodiment.

When the insertion portion 2 of the endoscope 1 is inserted into a body cavity and the bending portion 7 is bent, the up/down bending operation knob 13A is operated, for example. As a result, the pulley 21 rotates, to cause the down direction bending wire 22B which is one of the pair of bending operation wires 22 to move to the distal end side, and cause the up direction bending wire 22A which is the other of the pair of bending operation wires 22 to move to the proximal end side. That is, the bending portion 7 is bent in accordance with the pulling of the up direction bending wire 22A.

The range of each of the bending operation wires 22 where the powder lubricant 26 is applied on the coating portion 25 slidingly moves in the bending portion 7. On the other hand, the range of each of the bending operation wires 22 where the coating portion 25 is not provided and only the powder lubricant 26 is applied slidingly moves in each of the coil pipes 45.

The powder lubricant 26 applied to each of the bending operation wires 22 is fallen off by the each of the bending operation wires being slid as described above, but the powder lubricant 26 fallen off in each of the coil pipes 45, which is a part of the powder lubricant 26 applied to each of the bending operation wires 22, adheres to the inner circumferential surface of each of the coil pipes 45 and remains thereon.

As a result, when each of the bending operation wires 22 slides in the longitudinal direction, the presence of the powder lubricant 26 between the outer circumferential surface of each of the bending operation wires 22 and the inner circumferential surface of each of the coil pipes 45 reduces the frictional force to be generated. Therefore, the amount of force of the bending operation at the operation portion 3 is reduced.

When the bending portion 7 is bent, each of the bending operation wires 22 contacts the wire receiver 23 to receive a large load. However, the coating portion 25 is provided on the part of each of the bending operation wires 22, the part being arranged in the bending portion 7, and the powder lubricant 26 is applied on the surface of the coating portion 25. Therefore, when each of the bending operation wires 22 contacts the wire receiver 23 and receives a load therefrom, the frictional force to be generated is reduced by the applied powder lubricant 26 in the initial state. In addition, since the coating portion 25 of the predetermined thickness is provided, each of the wires 22 is not likely to receive a large load. In addition, the thickness of the coating portion 25 is set to the predetermined thickness, which prevents the coating portion 25 from being separated.

Therefore, cutting of the bending operation wires 22 due to the load applied from the wire receivers 23 is effectively prevented.

Note that it is necessary to apply the optimal powder lubricant 26 to the bending operation wires 22. Therefore, a plurality of kinds of power lubricants such as PTFE-based lubricant, silicone rubber-based lubricant, graphite-based lubricant, and graphite fluoride-based lubricant are prepared, to select the powder lubricant 26 to be applied to the bending operation wires 22 by performing a test.

The test for selecting the lubricant is performed such that each of the above-described powder lubricants is applied to the non-coated bending operation wire 22, the wire 22 to which the lubricant is applied is inserted in the coil pipe 45, the bending operation wire 22 is repeatedly moved slidingly, the wire pulling force is measured when the wire is moved slidingly, and differences in the wire pulling forces for each of the lubricants are compared with one another.

As a result of the test, compared with the wire pulling force when the graphite fluoride-based lubricant is applied, the wire pulling force is increased by 50% when the PTFE-based lubricant is applied, increased by 50 to 100% when the silicone rubber-based lubricant is applied, and increased by more than 100% when the graphite-based lubricant is applied. Therefore, the graphite fluoride-based lubricant, which shows the lowest sliding resistance, is applied to the bending operation wires 22 according to the present embodiment.

Thus, the lubricating property of the bending operation wires 22 in the bending portion 7 is ensured by the coating portion 25, and the lubricating property of the bending operation wires 22 in the coil pipes 45 is ensured by the powder lubricant 26, which enables the amount of the bending operation force to be reduced and prevents the cutting of the bending operation wires 22 caused by the load, by effectively using the two lubricants in combination.

Note that each of the sheaths is a closed coil in the above description. However, instead of the closed coil, each of the sheaths may be formed by a plurality of ring-shaped members aligned so that each member contacts with the adjacent member, pipe-shaped members aligned so that each member contacts with the adjacent member, or one pipe-shaped member.

In addition, in order to prevent the occurrence of such failure that the bare part of each of the bending operation wires 22 to which the coating is not applied is arranged in the bending portion 7 and cutting of each of the wires is caused, the coating portion 25 is configured such that the range to which the coating processing is applied is set under the condition that the bare part does not get out of the sheath even in the case where the down direction bending wire 22B is drawn into the bending portion 7 when the up direction bending wire 22A is pulled to bring the bending portion 7 into the maximum bent state in the up direction, for example.

Furthermore, as described above, when the coating portion 25 applied to each of the bending operation wires 22 slides in each of the coil pipes 45, the coating portion including the binder is likely to be peeled off, which may interfere with the forward or backward movement of each of the bending operation wires 22. Therefore, it is desirable to set the range to which the coating processing is applied to a minimum range that meets the above-described condition.

As result, occurrence of the failure that the bare part of the bending operation wire 22 to which the coating is not applied is arranged in the bending portion 7 and the wire is cut and occurrence of the failure caused by the coating portion 25 sliding in each of the coil pipes 45 are prevented.

Note that, in the present embodiment, an example in which the wires are bending operation wires 22 is shown. However, the wires may be raising wires inserted into the insertion portion of the endoscope and pulled for raising a raising stand that is provided at the insertion portion and configured to raise a treatment instrument inserted into the insertion portion.

Note that, in the present embodiment, an example in which the wires are bending operation wires 22 is shown. However, the wires may be transmission wires that transmit a driving force for rotating an insertion assisting portion around the longitudinal axis of the insertion portion, the insertion assisting portion being provided on the outer surface of the insertion portion and configured to move the insertion portion forward or backward with respect to the subject to be examined.

Note that, in the present embodiment, an example in which the wires are bending operation wires 22 is shown. However, the wires may be lens operation wires inserted in the insertion portion and pulled for moving a photographing lens that is provided in the insertion portion and configured to be movable in the optical axis direction.

The bending apparatus is not limited to an endoscope, but may be other kinds of devices such as an insertion assisting instrument, a treatment instrument used with the endoscope, and the like, other than the endoscope.

Note that the present invention is not limited only to the above-mentioned embodiment, but may be modified and embodied in various forms without departing from the gist of the present invention.

The present invention is capable of providing a bending apparatus that reduces an amount of operation force at the time of bending operation and preventing bending operation wires from being cut in a bending portion.

## Claims

1. An endoscope (1) including a wire device comprising:
a sheath (45) arranged inside said endoscope and
a wire (22) inserted in the sheath (45) and moved in an axial direction or rotated in the sheath,
wherein the wire (22) is configured such that processing for coating including a binder component is performed only on a range of the wire, the range being exposed from the sheath, and a powder lubricant which does not include the binder component is applied to at least a range of the wire, the range being inserted in the sheath.

2. The endoscope according to claim 1, wherein the sheath is made of resin.

3. The endoscope according to claim 1, wherein the powder lubricant is applied to the range of the wire where the coating processing is performed and the range of the wire where the coating processing is not performed.

4. The endoscope according to claim 1, wherein the powder lubricant is graphite fluoride.

5. The endoscope according to claim 1, further comprising:
an insertion portion to be inserted into a subject to be examined;
a bendable bending tube provided at the insertion portion,
wherein the wire is a bending operation wire inserted in the insertion portion and pulled for bending the bending tube.

6. The endoscope according to claim 1, wherein
the bending operation wire is configured by a pair of wires,
the pair of wires is coupled with the bending tube so as to cause the bending tube to bend in directions different from each other, and
the processing for coating is performed such that a range where the coating processing is performed is set so as to prevent the range where the coating processing is not performed from getting out of the sheath in a state where one of the pair of the wires is pulled to bring the bending tube into a maximum bent state, to thereby cause the pair of the bending wires to be drawn into the bending tube.

7. The endoscope according to claim 1, wherein the bending tube includes a bending piece group configured by rotatably coupling a plurality of bending pieces.

8. The endoscope according to claim 1, wherein the sheath is formed by spirally winding an element wire, and the bending operation wire is inserted so as to be able to move forward or backward in the sheath.

9. The endoscope according to claim 1, comprising:
an insertion portion to be inserted into a subject to be examined; and
a raising stand provided to the insertion portion and configured to raise a treatment instrument inserted in the insertion portion,
wherein the wire is a raising wire inserted in the insertion portion and pulled for performing raising operation of the raising stand.

10. The endoscope according to claim 1, comprising:
an insertion portion to be inserted into a subject to be examined; and
a photographing lens provided in the insertion portion and movable in an optical axis direction,
wherein the wire is a lens operation wire inserted in the insertion portion and pulled for moving the photographing lens.

11. The endoscope according to claim 1, comprising:
an insertion portion to be inserted into a subject to be examined; and
an insertion assisting portion provided on an outer surface of the insertion portion and configured to move the insertion portion forward or backward with respect to the subject to be examined,
wherein the wire is a transmission wire that transmits a driving force for rotating the insertion assisting portion around a longitudinal axis direction of the insertion portion.

## Patentansprüche

1. Endoskop (1), umfassend eine Kabelvorrichtung, die umfasst:
eine Hülle (45), die im Innern des Endoskops (1) angeordnet ist, und
ein Kabel, das in der Hülle (45) eingeführt ist und in eine Achsrichtung bewegt oder in der Hülle gedreht wird,
wobei das Kabel (22) derart ausgelegt ist, dass ein Prozess zur Beschichtung, die eine Bindemittelkomponente umfasst, nur auf einem Bereich des Kabels durchgeführt wird, wobei der Bereich von der Hülle freiliegt, und ein Pulverschmiermittel, das nicht die Bindemittelkomponente umfasst, auf mindestens einen Bereich des Kabels aufgetragen wird, wobei der Bereich in die Hülle eingeführt wird.

2. Endoskop nach Anspruch 1, wobei die Hülle aus Harz besteht.

3. Endoskop nach Anspruch 1, wobei das Pulverschmiermittel auf den Bereich des Kabels aufgetragen wird, auf dem der Beschichtungsprozess durchgeführt wird, und auf den Bereich des Kabels, auf dem der Beschichtungsprozess nicht durchgeführt wird.

4. Endoskop nach Anspruch 1, wobei das Pulverschmiermittel Graphitfluorid ist.

5. Endoskop nach Anspruch 1, ferner umfassend:
einen Einführabschnitt, der in ein zu untersuchendes Subjekt einzuführen ist;
ein biegbares Biegerohr, das an dem Einführabschnitt vorgesehen ist,
wobei das Kabel ein Bedienungsbiegekabel ist, das in dem Einführabschnitt eingeführt ist und zum Biegen des Biegerohrs gezogen wird.

6. Endoskop nach Anspruch 1, wobei
das Bedienungsbiegekabel durch ein Paar Kabel ausgelegt ist,
das Paar Kabel derart mit dem Biegerohr gekoppelt ist, dass bewirkt wird, dass sich das Biegerohr in voneinander verschiedene Richtungen biegt, und
der Beschichtungsprozess derart durchgeführt wird, dass ein Bereich, in dem der Beschichtungsprozess durchgeführt wird, derart eingestellt wird, dass verhindert wird, dass der Bereich, in dem der Beschichtungsprozess nicht durchgeführt ist, in einem Zustand aus der Hülle herauskommt, in dem ein Kabel des Kabelpaares gezogen wird, um das Biegerohr in einen maximalen Biegezustand zu bringen, um dadurch zu bewirken, dass das Paar Biegekabel in das Biegerohr gezogen wird.

7. Endoskop nach Anspruch 1,
wobei das Biegerohr eine Biegestückgruppe umfasst, die durch drehbares Koppeln einer Vielzahl von Biegestücken ausgelegt ist.

8. Endoskop nach Anspruch 1, wobei die Hülle durch spiralförmiges Wickeln eines Elementkabels gebildet ist, und das Bedienungsbiegekabel derart eingeführt ist, dass er in der Hülle vorwärts und rückwärts bewegt werden kann.

9. Endoskop nach Anspruch 1, umfassend:
einen Einführabschnitt, der in ein zu untersuchendes Subjekt einzuführen ist; und
ein Hebegestell, das an dem Einführabschnitt vorgesehen und dazu ausgelegt ist, ein in dem Einführabschnitt eingeführtes Behandlungsinstrument anzuheben,
wobei das Kabel ein Hebekabel ist, das in den Einführabschnitt eingeführt ist und zum Durchführen eines Hebevorgangs des Hebegestells gezogen wird.

10. Endoskop nach Anspruch 1, umfassend:
einen Einführabschnitt, der in ein zu untersuchendes Subjekt einzuführen ist; und
eine Fotografielinse, die in dem Einführabschnitt vorgesehen ist und in eine Richtung der optischen Achse bewegt werden kann,
wobei das Kabel ein Linsenbedienungskabel ist, das in dem Einführabschnitt eingeführt ist und zum Biegen der Fotografielinse gezogen wird.

11. Endoskop nach Anspruch 1, umfassend:
einen Einführabschnitt, der in ein zu untersuchendes Subjekt einzuführen ist; und
einen Einführhilfsabschnitt, der an einer Außenfläche des Einführabschnitts vorgesehen und dazu ausgelegt ist, den Einführabschnitt mit Bezug auf das zu untersuchende Subjekt vorwärts und rückwärts zu bewegen,
wobei das Kabel ein Sendekabel ist, das eine Antriebskraft zum Drehen des Einführhilfsabschnitts um eine Längsachsenrichtung des Einführabschnitts sendet.

## Revendications

1. Endoscope (1) comprenant un appareil à fil comprenant :
une gaine (45) disposée à l'intérieur dudit endoscope (1), et
un fil (22) inséré dans la gaine (45) et déplacé dans une direction axiale ou tourné dans la gaine,
le fil (22) étant configuré de telle sorte qu'un traitement de revêtement comprenant un composant liant est réalisé uniquement sur une longueur du fil, la longueur étant exposée à partir de la gaine, et un lubrifiant en poudre qui ne comprend pas le composant liant est appliqué sur au moins une longueur du fil, la longueur étant insérée dans la gaine.

2. Endoscope selon la revendication 1, dans lequel la gaine est faite de résine.

3. Endoscope selon la revendication 1, dans lequel le lubrifiant en poudre est appliqué sur la longueur du fil où le traitement de revêtement est réalisé et sur la longueur du fil où le traitement de revêtement n'est pas réalisé.

4. Endoscope selon la revendication 1, dans lequel le lubrifiant en poudre est du fluorure de graphite.

5. Endoscope selon la revendication 1, comprenant en outre :
une partie d'insertion à insérer dans un sujet à examiner ;
un tube de courbure apte à être courbé, disposé au niveau de la partie d'insertion,
le fil étant un fil d'actionnement de courbure inséré dans la partie d'insertion et tiré pour courber le tube de courbure.

6. Endoscope selon la revendication 1, dans lequel le fil d'actionnement de courbure est configuré par une paire de fils,
la paire de fils est couplée au tube de courbure de façon à amener le tube courbure à se courber dans des directions différentes les unes des autres, et
le traitement de revêtement est réalisé de telle sorte qu'une longueur où le traitement de revêtement est réalisé est définie de façon à empêcher la longueur où le traitement de revêtement n'est pas réalisé de sortir de la gaine dans un état dans lequel l'un parmi la paire de fils est tiré pour amener le tube de courbure dans un état courbé maximal, afin d'amener la paire de fils de courbure à être tirée dans le tube de courbure.

7. Endoscope selon la revendication 1, dans lequel le tube de courbure comprend un groupe d'organes de courbure configuré par couplage rotatif d'une pluralité d'organes de courbure.

8. Endoscope selon la revendication 1, dans lequel la gaine est formée par enroulement en spirale d'un fil individuel, et le fil d'actionnement de courbure est inséré de façon à être apte à se déplacer vers l'avant ou vers l'arrière dans la gaine.

9. Endoscope selon la revendication 1, comprenant :
une partie d'insertion à insérer dans un sujet à examiner ; et
un socle d'élévation situé sur la partie d'insertion et configuré pour élever un instrument de traitement inséré dans la partie d'insertion,
le fil étant un fil d'élévation inséré dans la partie d'insertion et tiré pour réaliser une opération d'élévation du socle d'élévation.

10. Endoscope selon la revendication 1, comprenant :
une partie d'insertion à insérer dans un sujet à examiner ; et
une lentille de photographie située dans la partie d'insertion et mobile dans une direction d'axe optique,
le fil étant un fil d'actionnement de lentille inséré dans la partie d'insertion et tiré pour déplacer la lentille de photographie.

11. Endoscope selon la revendication 1, comprenant :
une partie d'insertion à insérer dans un sujet à examiner ; et
une partie d'aide à l'insertion située sur une surface externe de la partie d'insertion et configurée pour déplacer la partie d'insertion vers l'avant ou vers l'arrière par rapport au sujet à examiner,
le fil étant un fil de transmission qui transmet une force d'entraînement pour faire tourner la partie d'aide à l'insertion autour d'une direction axiale longitudinale de la partie d'insertion.
